# EUROPEAN PATENT APPLICATION

(11) **EP 4 531 500 A1**
(43) Date of publication of application: **02.04.2025**
(21) Application number: 24203341.3
(22) Date of filing: 27.09.2024
(51) Int. Cl.: H05K 1/02, H05K 1/16, H05K 3/00, H05K 3/34, H05K 13/08, H05K 13/00, A61B 5/00, H05K 1/18, H05K 3/32

(54) **SYSTEM AND METHOD TO CONTROL HEIGHT BETWEEN A JOINING TOOL AND A CONNECTION PAD**

(30) Priority: 29.09.2023 US 202363541308 P; 20.09.2024 US 202418890860
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); SITNITSKY, Ilya, 2066717 Yokneam (IL); SHECHTMAN, Alexander, 2066717 Yokneam (IL); FUCHS, Amit, 2066717 Yokneam (IL)
(74) Representative: Carpmaels & Ransford LLP

(57) **Abstract**

A method of electrically connecting a wire to a connection pad of a flexible circuit strip using a joining tool, which method comprising: receiving the flexible circuit strip on a support surface, wherein the flexible circuit strip includes a plurality of electrodes, a plurality of connection pads and conductive traces connecting each of the plurality of electrodes to a connection pad of the plurality of connection pads; positioning a joining tool over a first connection pad of the flexible circuit strip; monitoring a first capacitance between said joining tool and a conductive layer positioned under the connection pads, wherein said conductive layer is sized, shaped, and positioned to extend over a footprint of said plurality of connection pads; controllably lowering said joining tool toward said first connection pad until the first capacitance reaches a first predefined capacitance threshold, wherein said first predefined capacitance threshold defines a first height of said joining tool with respect to said flexible circuit strip; monitoring a second capacitance between a first electrode electrically connected to said first connection pad and said joining tool; controllably lowering said joining tool from said first height until said second capacitance reaches a second predefined capacitance threshold, wherein said second predefined capacitance threshold defines a second height of said joining tool with respect to said first connection pad; and activating said joining tool to electrically connect said first connection pad to said wire.

## Description

### TECHNOLOGICAL FIELD

The present disclosure, in some embodiments thereof, relates to catheter manufacture and, more particularly, but not exclusively, to establishing electrical connections to flexible circuit strips of the catheter.

### BACKGROUND ART

A wide range of medical procedures involve placing probes, such as catheters, within a patient's body. Electrode catheters have been in common use in medical practice for many years. They are used to stimulate and map electrical activity in the heart and to ablate sites of aberrant electrical activity.

Location sensing systems have been developed for tracking such probes. Magnetic location sensing is one of the methods known in the art. In magnetic location sensing, magnetic field generators are typically placed at known locations external to the patient. A magnetic field sensor within the distal end of the probe generates electrical signals in response to these magnetic fields, which are processed to determine the coordinate locations of the distal end of the probe. Locations may also be tracked using impedance or current based systems.

A wall cavity of an organ of a patient, such as a cardiac cavity, can be mapped and/or ablated using a catheter having multiple electrodes fitted at an expandable distal end assembly of the catheter. The expandable distal-end assembly is coupled at a distal end of a catheter shaft for insertion into the cavity. The expandable distal end assembly may be shaped in the form of a balloon, basket and/or another type of cage and may include a plurality of electrodes configured for sensing and/or delivering therapeutic signals.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
FIG. 1A is a schematic view of a basket catheter according to some embodiments of the disclosure;
FIG. 1B is a simplified schematic of flexible polymer circuit strips, according to some embodiments of the disclosure;
FIG. 2A is a simplified schematic of a system for providing electrical connections to a flexible circuit strip, according to some embodiments of the disclosure;
FIGs. 2B and 2C are simplified schematics illustrating attaching of a connection to a pad, according to some embodiments of the disclosure;
FIG. 3 is a method of establishing a connection to a flexible circuit strip, according to some embodiments of the disclosure;
FIG. 4 is a simplified schematic theoretical plot of capacitance with time, according to some embodiments of the disclosure; and
FIG. 5 is a simplified schematic theoretical plot of capacitance with time, according to some embodiments of the disclosure.In some embodiments, although non-limiting, like elements are referred to using like numerals.

### DETAILED DESCRIPTION OF EMBODIMENTS

The present disclosure, in some embodiments thereof, relates to catheter manufacture and, more particularly, but not exclusively, to establishing electrical connections to flexible circuit strips of the catheter.

### Overview

Some catheters, such as basket catheters, may have about 100 electrodes mounted on a plurality of splines of the basket, which is coupled through a shaft of the catheter to a connector at the proximal end of the shaft. Both a printed circuit board (PCB), also herein termed "flexible circuit strip" on the splines and a PCB of the connector have pads and a plurality of wires, e.g., a wire for each electrode, running through the shaft provides electrical coupling. The distal end of each wire is coupled to a predefined pad on the spline, and a proximal end of each wire is coupled to a corresponding pad in the connector, so as to exchange electrical signals between the electrodes and a control console, e.g., a PIU (patient interface unit) of the catheter, via the shaft. The large number of pads and wires may require scaling down the pad size to tens of microns. The pad size may be in the order of magnitude of tens of micrometers, also referred to herein as microns) of each pad on the printed circuit board, resulting in a time-consuming and costly coupling process between the wires and the respective pads.

A broad aspect of some embodiments of the disclosure relates to an automated process for soldering or welding wires to pads (also herein termed "connection pads") of a flexible circuit strip of a catheter device by automated positioning of a joining tool. In some embodiments, the flexible circuit strip includes a plurality of pads, where electrical connections are individually established to the individual pads (e.g., sequentially) by bringing a joining tool towards each pad e.g., into contact with each pad. Positioning of the joining tool (e.g. height of the tool tip above a pad) may be controlled using measurement of capacitances at the joining tool.

A potential benefit of automation and/or automation using capacitance measurement/s being the ability to rapidly and/or accurately establish connections to small sized pads which may be densely disposed on a small surface area of a flexible circuit strip.

In some embodiments, the flexible circuit strip and/or pads are delicate (e.g., mechanically delicate), for example, due to their size. The sizing potentially meaning that it is difficult to accurately position the joining tool at a correct height above each pad without damaging the pad and/or flexible circuit strip but while the height of the tool above this pad is sufficient that joining performed with the tool makes a sound connection (e.g., sufficiently electrically conductive and/or mechanically strong).

A potential benefit of accurately bringing the joining tool to the pad is reduced risk of damage to the flexible circuit strip, for example, by application of excessive pressure by the joining tool. A further potential benefit of accurately brining the joining tool to the pad is the ability to make high quality (e.g., mechanically strong and/or electrically conductive) connections to the pads, for example, as associated with the joining tool being correctly positioned with respect to the pad. Where correct positioning includes accurately positioning a tip of the joining tool at a defined distance from (e.g., height above) the external surface of the pad to be connected to. In an exemplary embodiment, a desired distance (e.g., height) between an outer surface of the pad and a tip of the joining tool is at the order of microns.

An aspect of some embodiments of the disclosure relates to automated positioning of the joining tool using a multi-step process and/or a plurality of different measurements.

Where, optionally, the joining tool is brought into a general location of the pad, using one or more image acquired by a camera (e.g., visible light camera). In some embodiments, positioning of the joining tool using imaging is in both a height direction generally perpendicular to planar surface/s of the flexible circuit strip (also herein termed a "z" direction) above the pad and in a lateral direction (e.g., also herein termed an "x-y" direction) generally parallel to the flexible circuit strip (e.g., planar surface/s of the flexible circuit strip).

In some embodiments, for example, after general positioning of the joining tool using image/s, capacitance measurement/s are used to bring the joining tool into position above the pad e.g., into contact with the pad.

In some embodiments, a first capacitance is measured between the joining tool and a conductive element located with the flexible circuit strip. Where, in some embodiments, the conductive element is close to (e.g., within a 1mm of) and/or in contact with and/or forms part of the flexible circuit strip, for example, the conductive element being a conductive layer. The pads may be disposed spatially between the joining tool (e.g., after general positioning of the joining tool) and the conductive element, movement of the joining tool towards the conductive element reducing a distance (e.g., height) between the pad and joining tool. For example, where the conductive element is disposed extending underneath the pads, the pads disposed between the conductive element and the joining tool which may approach the pads from above. In some embodiments, the conductive element (e.g., conductive layer) overlaps a region of the pads, for example, extending underneath an area (e.g., footprint) of the flexible circuit strip hosting the pads e.g., extending under the entire footprint of the pads.

Although description herein is regarding a joining tool approaching pads from above it should be understood that this disclosure also relates to a situation where the flexible circuit strip is orientated at different positions and/or angles with respect to the joining tool, for example, where the flexible circuit strip is disposed above the joining tool e.g., the joining tool moving upwards to approach the pads.

In some embodiments, the conductive element extends generally parallel to the pads e.g., underneath the pads. In some embodiments, the conductive element is disposed within a body of the flexible circuit strip. In some embodiments, the conductive element is located underneath the flexible circuit strip e.g., where the conductive element supports the flexible circuit strip (e.g., is mounted to or part of a support) and/or where the conductive element is attached to a back side of the flexible circuit strip (the back side a surface of the flexible circuit strip opposing a top side of the flexible circuit strip hosting the pads).

In some embodiments, the flexible circuit strip includes a plurality of pads each pad connected to a corresponding electrode by an electrical connector (e.g., a wire and/or conductive trace, where the term wire as used in this context should be understood to encompass a "conductive trace" or "trace") which extends extend along (e.g., through) a body of the flexible circuit strip. In some embodiments, pads are disposed at a first portion of the flexible circuit strip (e.g., proximal end of the flexible circuit strip) and are connected to sensing electrodes located at a second portion of the flexible circuit strip (e.g., distal end of the flexible circuit strip).

In some embodiments, the conductive element overlaps at least one of the plurality of pads, or a subset of the plurality of pads of the flexible circuit strip, or overlaps all of the pads (e.g., extending underneath all of areas hosing the pads). In some embodiments, the conductive element may not extend continuously underneath all of an area hosting the pads, but may extend underneath each individual pad with connecting portions therebetween.

For example, in embodiments were the conductive element overlaps a subset of the plurality of pads, measurements acquired (and/or control signals generated using the measurements) concerning a first pad which is overlapped the conductive elements may be used in connection of a subsequent bad which is not overlapped by the conductive element.

In some embodiments, the conductive element also overlaps (e.g., extends underneath) a portion of the flexible circuit strip hosting electrodes. Where, in some embodiments, the conductive element extends over an entire footprint of the pads and electrodes.

In some embodiments, a second capacitance is measured between the pad itself (and/or a direct electrical connection to the pad) and the joining tool. In some embodiments, the direct electrical connection is provided by an electrode of the flexible circuit strip electrically which is connected to the pad.

In an exemplary embodiment, the first capacitance is used to move the joining tool closer (e.g., than that achieved using imaging) to the pad, and the second capacitance is used for fine positioning of the joining tool with respect to the pad e.g., prior to activating the joining tool to establish an electrical connection to the pad.

In some embodiments, a surface of the conductive element opposing the joining tool has a larger surface area than that of each pad. Where the larger surface area and/or higher dielectric constant of the material of the flexible circuit strip (e.g., as compared to air between the joining tool and the pad) may provide higher capacitance measurements e.g., than the second capacitance measurement between the joining tool and pad.

In some embodiments, pads are disposed at different distances (heights) from the joining tool. For example, associated with flexibility of the strip e.g., the strip does not conform fully to a flat support surface. For example, where, in some embodiments, different distances from the joining tool of pads may be associated with different topologies of the pad/s and/or top surface of the flexible circuit strip. For example, where the flexible circuit strip is mounted to a support structure prior to establishing electrical connections to the pads and the support structure (e.g., a nitinol spline) may not form a planar support to the flexible circuit strip (e.g., the nitinol spline may have variable thickness).

In some embodiments, the pads have a small connection surface are and/or are positioned close to one another in comparison to size and/or spacing of electrodes. Contacting of the electrode for measurement of the second capacitance potentially being easier and/or providing better electrical contact for measurements.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary catheter

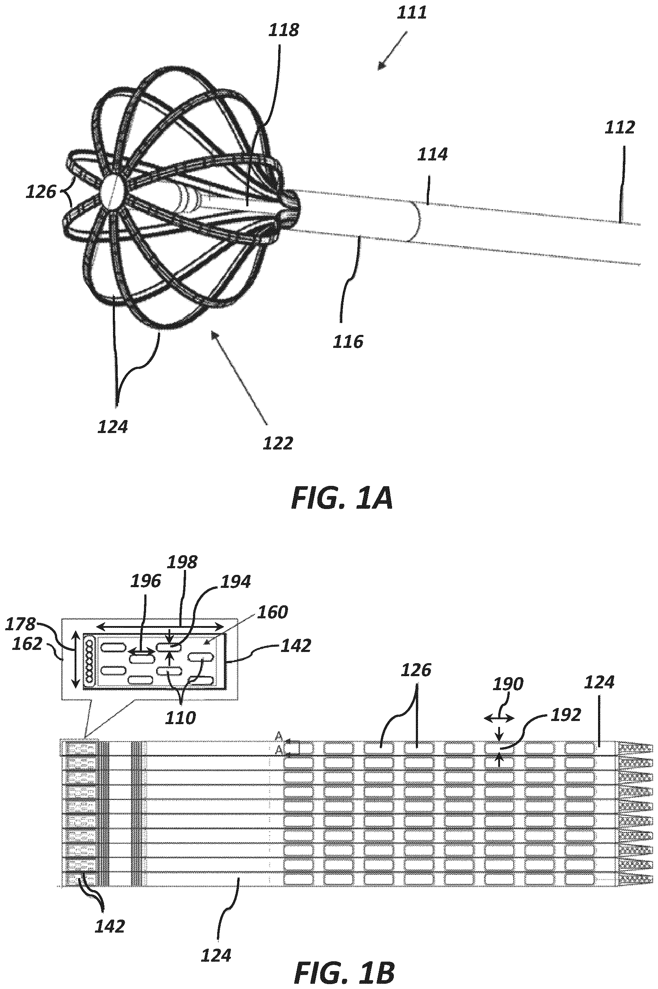

FIG. 1A is a schematic view of a basket catheter 111 according to some embodiments of the disclosure.

The basket catheter 111 includes an elongated deflectable element 112 having a distal end 114, a coupler 116 connected to the distal end 114, and an optional pusher 118. The optional pusher 118 is configured to be advanced and retracted through the deflectable element 112, for example, using a manipulator or handle (not shown). The basket catheter 111 also includes an expandable assembly 122 comprising a plurality of flexible circuit strips 124 (only some labeled for the sake of simplicity) also herein termed "flexible splines". Where, in some embodiments, the flexible circuit strips 124 are polymer circuit strips. Each flexible circuit strip 124 includes multiple electrodes 126 disposed thereon (only some labeled for the sake of simplicity). The formation of the various elements and how they are connected with each other are described in more detail in US Patent Application Publication No. US 2021/0187241.

In some embodiments, form and/or structure of basket catheter 111 is provided by a basket structure, which in some embodiments, includes (e.g., is formed of) nitinol e.g., including a plurality of nitinol strips (also herein termed "splines"). Where a least a portion of flexible circuit strips 124 are each supported by a corresponding nitinol strip. In some embodiments, strips of the nitinol basket structure are connected together e.g., at a proximal end of basket catheter 111. Flexible circuit strips 124 may be mounted to the nitinol basket structure prior to or after establishing electrical connections to the pads (e.g., pads 110 FIG. 1B) of flexible circuit strips 124. In some embodiments, electrical connections to pads 110 of flexible circuit strips 124 are established when the flexible circuit strips 124 are in a flat and/or un-disconnected configuration e.g., the configuration illustrated in FIG. 1B, prior or after connecting of the flexible circuit strips to the nitinol support structure.

### Exemplary flexible circuit strip embodiments

FIG. 1B is a top view of flexible polymer circuit strips 124 prior to their assembly for use in the basket catheter of FIG. 1A.

Reference is now made to FIG. 1B, which is a schematic view of the flexible polymer circuit strips 124 for use in the basket catheter 111 of FIG. 1A. The flexible polymer circuit strips 124 may be formed from a single piece of polymer, such as polyimide. Circuit strips 124 may be connected to each other by polyimide, or assembled as individual pieces that are held in proper alignment and secured to coupler 116 FIG. 1A. By manufacturing circuit strips 124 as individual components the yield of the base circuit may be increased as a failed electrode scraps one circuit strip rather than an entire assembly of strips. Respective first ends 142 of the respective flexible polymer circuit strips 124 include an electrical connection array 160.

An inset 162 shows that the electrical connection array 160 includes pads 110 thereon (only some labeled for the sake of simplicity). The pads 110 are connected via wires (not shown), optionally located on the back of the flexible polymer circuit strips 124, to respective ones of the electrodes 126 disposed on the front of the flexible circuit strips 124. Away from the region of the first ends 142, the flexible circuit strips 124 are separate from each other to allow the flexible circuit strips 124 to form the expandable assembly 122 (FIG. 1A) when connected to the basket catheter 111. Wires (not shown) may connect the electrodes 126 to control circuitry (not shown) via pads 110. The wires may be disposed in lumens (not illustrated) of the elongated deflectable element 112 (FIG. 1A).

The flexible circuit strips 124 may have any suitable dimensions. For example, the length of the flexible circuit strips 124 may be in the range of 10 mm to 60 mm, e.g., 30 mm, the width of the flexible circuit strips 124 may be in the range of 0.25 mm to 3 mm, e.g., 0.72 mm, the thickness of the flexible circuit strips 124 may be in the range of 0.005 mm to 0.14 mm.

In some embodiments, electrodes 126 extend to occupy a majority of the width of the flexible circuit strips 124 by which they are hosted. Where, in some embodiments, electrode width 192 is 0.25-3 mm, and/or electrode length is 0.25-3 mm.

In some embodiments, one or more pad dimensions are smaller than electrode dimensions. Where the surface area the pad presents for contact (e.g., by a measurement probe or joining tool) is smaller than that presented by the electrodes. For example, a pad upper surface area being 1-20% of that of the electrodes. For example, a pad width 194 being 0.05-0.5 mm, and/or a pad length 196 0.1-1 mm.

A surface area 162 of the flexible circuit strips 124 on which pads 142 are disposed may be small and/or the density of pads on the area may be high. For example, the area having a width 178 of 0.25-3 mm and a length 198 of 0.5-6 mm and/or the proportion of the surface area occupied by pads is 20-50%.

### Exemplary electrical connection system

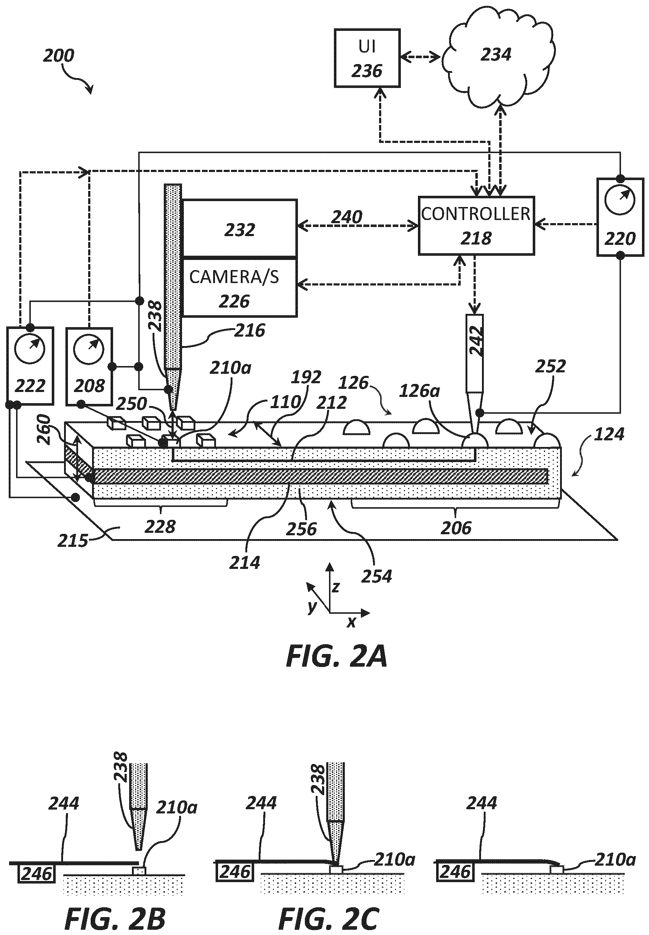

FIG. 2A is a simplified schematic of a system 200 for providing electrical connections to a flexible circuit strip 124, according to some embodiments of the disclosure.

In some embodiments, flexible circuit strip 124 is a portion of a catheter e.g., having one or more features as illustrated in and/or described regarding catheter 111 FIG. 1A. Where, in some embodiments, flexible circuit strip 124 corresponds to one or more (e.g., each) of the flexible circuit strips 124 of FIG. 1A and/or FIG. 1B.

In some embodiments, system 200 includes a joining tool 216 configured to join an electrical connector e.g., a wire (not illustrated) to pads 110 (e.g., one pad at a time). In some embodiments, joining tool 216 is a heat joining tool e.g., soldering iron, or welding gun, or ultrasonic joining tool.

In some embodiments, system 200 includes a support 215 which provides mechanical support to flexible circuit strip 124. For example, support 215 holding flexible circuit strip 124 in position potentially enabling joining tool 216 to apply pressure to a pad e.g., during a joining process. In some embodiments, support 215 is rigid.

In some embodiments, system 200 includes one or more sensor 208, 220, 222 where one or more of sensors 208, 220, 222 are capacitance sensors.

In FIG. 2A, in some embodiments, electrical connections (e.g., for capacitance measurements) are illustrated by solid lines, and data connections are illustrated by dashed lined arrows.

In some embodiments, flexible circuit strip 124 has at least one (e.g., a plurality) of electrodes 126. Where electrodes 126 may be disposed on a distal portion 206 of flexible circuit strip 124. Electrodes 126 may be disposed on an external surface of flexible circuit strip 124 e.g., one or more of electrodes 126 may protrude from a generally planar external surface (e.g., a top surface) of flexible circuit strip 124 and/or one or more of electrodes 126 may have an electrode top surface flush (or recessed from) the flexible circuit strip 124 top surface.

In some embodiments, a plurality of pads 110 are disposed on a first (e.g., proximal) portion 228 of flexible circuit strip 124 and a plurality of electrodes 126 are disposed on a second (e.g., distal) portion 206 of flexible circuit strip 124. In some embodiments, one or more of electrodes 126 e.g., each electrode, is electrically connected to a corresponding pad of plurality of pads 110 e.g., each electrode to a pad by an individual wire. In some embodiments, the electrical connections (e.g., wires) extend through a body of flexible circuit strip 124 e.g., where in FIG. 2A a single such electrical connection wire 212 is illustrated connecting a specific electrode 126a to a specific pad 210a.

In some embodiments, flexible circuit strip 124 includes a plurality of layers where, for example, wires connecting pads 110 to electrodes 126 are sandwiched between at least two layers. Where, in some embodiments, an outer surface 252 of flexible circuit strip 124 (also herein termed "top surface" 252) hosts pads 110 and electrodes 126. In some embodiments, top surface 252 includes electrically insulating material (e.g., portions which are not the pads or electrodes are formed of polymer).

In some embodiments, flexible circuit strip 124 includes a conductive element 214 which extends underneath (e.g., generally parallel to) one or more of plurality of pads 110 e.g., to each of pads 110 or to a subset of pads 110. In some embodiments, conductive element 214 extends underneath at least one of the plurality of pads 110. In some embodiments, conductive element 214 extends underneath all of the plurality of pads 110. In some embodiments, conductive element extends underneath at least one pad and at least one electrode. In some embodiments, conductive element 214 extends underneath one or both of the plurality of electrodes 126 and the plurality of pads 110. In an exemplary embodiment, conductive element 214 extends underneath each of the plurality of electrodes 126 and each of the plurality of pads 110.

Conductive element 214, in some embodiments, extends underneath plurality of pads 110 (and, optionally electrodes 126) where a shortest distance measured between each pad (and optionally electrode) and the conductive element is the same e.g., where the distances have at most 5% variation (percentage e.g., determined as a percentage of an average of distances measured between the pads (and optionally electrodes) and the conductive elements).

In some embodiments, conductive element 214 extends along flexible circuit strip 124 being, in some embodiments, generally parallel to top surface 252 and/or base surface 254. In some embodiments, conductive element 214 extends laterally (e.g., perpendicular to a direction of elongation of elongate flexible circuit strip 124) across at least majority (e.g., across at least 80%, or all as illustrated in FIG. 2A) of a width 192 of flexible circuit strip.

In some embodiments, conductive element 214 is a layer of the layered structure of flexible circuit strip 124. Where, in some embodiments, insulating layer/s separate conductive element 214, pads 110 and electrodes 126. Optionally, for example, as illustrated in FIG. 2A, conductive element 214 is an inner layer of flexible circuit strip 124 where a layer 256 (e.g., electrically insulating) is disposed between conductive element 214 and a base surface 254 of flexible circuit strip 124. Where base surface 254 of flexible circuit strip 124 is a generally planar surface opposing the top surface. Alternatively, in some embodiments, conductive element 214 forms at least a portion of the bottom surface of flexible circuit strip 124 e.g. layer 256 being absent.

Alternatively or additionally, (e.g., to conductive element 214), in some embodiments, support 215 includes conductive material and provides the conductive element extending underneath electrodes 126 and/or pads 110 (e.g., in contact with) the base surface of the flexible circuit strip.

In some embodiments, joining tool 216 is automatically moveable with respect to flexible circuit strip 124 (e.g., by one or more actuators/s) for moving the joining tool between locations of the plurality of pads. In some embodiments, relative movement of the joining tool and flexible circuit strip is in three directions. The directions including, for example, a direction generally perpendicular to surfaces 252, 254, also termed a "z-direction" and/or in a lateral distance, generally parallel to surfaces 252, 254, also herein termed a movement in a "x-y direction". In some embodiments, a minimum distance between the joining tool (e.g., the joining tool tip) and a pad is referred to using these two directions, where a "height" is measured as a shortest distance in the z-direction between the joining tool and the pad outer surface and where a "lateral distance" is measured as a shortest distance in the x-y direction between the joining tool and a center of the pad outer surface and/or an external edge of the pad.

In some embodiments, the joining tool 216 is mounted on and/or is part of (e.g., is an end effector) of a robotic arm 232. Where actuator/s of the robotic arm are configured to move joining tool 216 in one or more dimension. Alternatively, in some embodiments, the joining tool 216 is mounted on and/or part of a stage 232 configured to move the joining tool in one or more direction. In some embodiments, support 215 is a part of and/or is held by a stage configured to move the flexible circuit strip with respect to the joining tool in one or more direction. In some embodiments, joining tool stage 232 is a z-stage configured to move the joining tool in the z direction e.g., control height of the joining tool above a pad. In some embodiments, the support stage is an x-y stage configured to move support 215 (and the flexible circuit strip) in the x-y plane (laterally). Movements of the joining tool and support 215 may be simultaneous or sequential. In some embodiments, robotic arm/stage 232 and/or support stage are configured for precision positioning e.g., able to control position at a range of microns e.g. to within 1-500 µm.

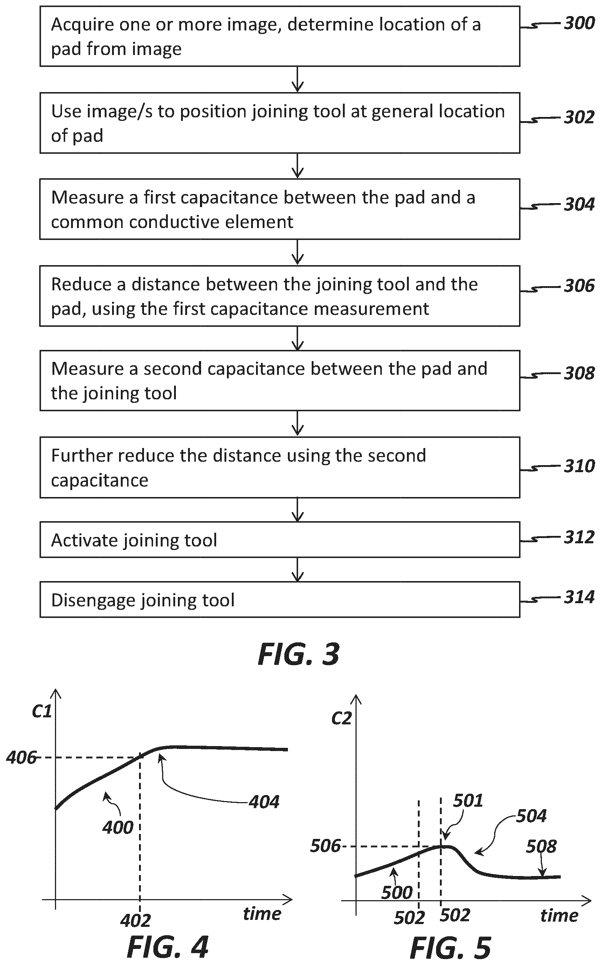

In some embodiments, position of the joining tool (e.g., as controlled by the robotic arm and/or stage/s) is controlled by a controller 218 which receives sensor signals (also herein termed "measurement signals" and/or "measurements") from one or more sensor. For example, from one or more of capacitance sensor/s 220, 222, 208. The controller may also receive image/s from one or more camera 226. The controller, for example, based on image/s and/or capacitance measurements received (e.g., as described in and/or regarding FIG. 3) generates and/or sends control signals (e.g., to one or more stage and/or robotic arm) for movement of joining tool 216 and/or flexible circuit strip 124 (e.g., specifically movement of a tip 228 of the joining tool). In some embodiments, controller 218, for example, based on image/s and/or capacitance measurements received generates control signals for timing and/or control of activation of joining tool 216. Where, in some embodiments, (e.g., where the joining tool includes a soldering iron and/or welding gun) activation includes heating the joining tool e.g., where control signals may include duration of heating of the joining tool and/or power driving heating of the joining tool and/or desired temperature of the tool. In some embodiments, system 200 includes one or more temperature sensor (not illustrated), for example, the joining tool may include a temperature sensor which, optionally, may provide temperature feedback measurement/s to controller 218 for control of temperature of the joining tool.

In some embodiments, the joining tool tip 238 includes an outer surface e.g., a cover including material which is highly electrically and/or thermally conductive and/or heat resistant. For example, the joining tool including a cover configured to cover (e.g., surround) at least a portion of joining tool 216 between a region of electrical contact between capacitance sensor/s 208, 220 and joining tool 216. In some embodiments, (e.g., as associated with high electrical and/or thermal conductivity) resistive and/or capacitive properties of the joining tool have low dependence on temperature.

In some embodiments, at least a portion of the joining tool which contacts a pad (e.g., tip 238 and/or a portion of the tip) during establishing connection to the pad is non-adhesive to material of pads 110. For example, at temperatures employed during establishing of connections to pads. When the joining tool is a soldering iron, material of the joining tool which contacts solder may be non-adhesive to solder e.g., alternatively or additionally to being non-adhesive to the pad surface.

In some embodiments, the joining tool tip and/or cover and/or region of the joining tool in an electrical path between the joining tool and sensor/s 208, 220 includes (e.g., is formed of) gold.

FIGs. 2B-C are simplified schematics illustrating attaching of a connection 244 to a pad 210a, according to some embodiments of the disclosure.

Where, FIG. 2B, in some embodiments, illustrates joining tool tip 238 approaching pad 210a. FIG. 2C, in some embodiments illustrates contact between pad 210a wire 244, and tip 238. FIG. 2D, in some embodiments, illustrates wire 244 in position connected to pad 210a.

When joining tool 216 includes a soldering iron, contact may additionally or alternatively be between the joining tool and solder. Solder may be supplied by the joining tool itself (e.g., joining tool 216 supplying soldier to tip 228). Alternatively or additionally, wire 244 may include solder and/or pad 210a may include solder where proximity to tip 228 may melt the connector and/or pad solder to connect the connector to the pad.

In some embodiments, wire 244 is positioned at pad 210a manually and/or automatically.

For example, where, in automated positioning of wire 244, wire 244 may be positioned at pad 210a by one or more actuator/s 246. Where, optionally, controller 218 controls position of wires 244 to be connected to the pad e.g., via actuator/s 246.

For example, where, in some embodiments, wire/s 244 (e.g., one wire per pad) are positioned (e.g., along with flexible circuit strip 124). For example, by adhering the wires to support 215. For example, by coupling wires 244 to a jig 246 configured to hold the wires 224 and optionally the flexible circuit strip in position with respect to each other (e.g., the jig including attachment and/or positioning means between support 215 and wires 224).

Alternatively, in some embodiments, joining tool 216 itself positions wire 244 for connection thereof at pad 210a. For example, stage/s and/or robotic arm 232 may be configured to position both the joining tool and the wire. Where, in some embodiments, wires for different pads may be fed to the joining tool e.g., sequentially.

Returning now to FIG. 2C, in some embodiments, system 200 includes a data connection 240 between controller 218 and actuator/s 232. Where data may flow in both directions. For example, in some embodiments, actuator signal/s and/or actuator sensor/s providing feedback to controller 218.

In some embodiments, a first capacitance is measured between conductive element 214 and joining tool 216. For example, by a capacitance sensor 222 configured to measure capacitance between conductive element 214 and joining tool 216 (e.g., a tip 238 of joining tool 216).

In some embodiments, a second capacitance is measured, e.g., by a sensor 220, between a flexible circuit strip electrode 126a and joining tool 216 (e.g., joining tool tip 238). Where the electrode is, in some embodiments, connected for measurement by contacting a probe 242 to the electrode e.g., as illustrated in FIG. 2A.

In some embodiments, alternatively or additionally, the second capacitance is measured between circuitry connected to an electrode and/or a pad (e.g., between connector 212) and joining tool 216. e.g., by a sensor 208,

In some embodiments, user instructions are received through and/or information is displaced to a user through one or more user interface (UI) 236. Where, in some embodiments, data is transferred between UI 236 and controller 218. For example, in some embodiments, user instructions are transferred by UI 236 to controller 218 which, in some embodiments, controls operation of system 200 based on the user instructions. One or more UI may be local to joining tool 216 and/or other system components and/or one or more UI may be located remotely.

Optionally, in some embodiments, external processing and/or memory circuitry 234 communicates with UI 236 and/or controller 218. For example, circuitry 234 enabling remote control of system 200 e.g., via remote UI/s.

### Exemplary electrical connection method

FIG. 3 is a method of establishing a connection to a flexible circuit strip, according to some embodiments of the disclosure.

At 300, optionally, in some embodiments, one or more image is acquired. Where, in some embodiments, the image is processed to identify pads, select a pad, and determine a position (e.g., with respect to the joining tool) of the selected pad.

Image/s may be processed to determine a spatial relationship between the joining tool and the pad e.g., where both the joining tool and the pad appear in acquired image/s.

Alternatively or additionally, image/s may be processed to determine a spatial relationship between the camera and selected pad, a known spatial relationship between the camera and joining tool, for example, being used to determine the spatial relationship between the joining tool and the pad.

At 302, optionally, in some embodiments, based on the spatial relationship determined at step 300 using image/s, the joining tool is positioned at a general location of pad (where the joining tool and/or the pad are moved), e.g., an XY stage is used to adjust positioning of one or more of the joining tool and the support surface on which the flexible circuit strip is disposed.

In some embodiments, movement/s of the joining tool towards the selected pad (and/or vice versa) are in more than one direction, for example, where the joining tool is moved laterally and in height above the flexible circuit strip and/or a pad. Movements in different directions may be concurrent or sequential.

In some embodiments, using images, the joining tool is positioned for example, to within a lateral distance from the center and/or an external edge of the pad of 1-50 mm, or 1-20 mm, or 2-10 mm, or about 5mm.

In some embodiments, using images, the joining tool is positioned for example, to within a height above the pad of 1-50 mm, or 1-20 mm, or 2-10 mm, or about 5mm.

Where actuator/s of stage/s and/or a robotic arm may be instructed (e.g., by controller 218) to position the joining tool and/or flexible circuit strip (e.g., by moving support 215), based on the determined spatial relationship. In some embodiments, steps 300 and 302 occur, at least partially, during a same time period. For example, where images are acquired during movement of the joining tool, the images being used to provide feedback for control of movements of the joining tool.

In some embodiments, the joining tool is positioned in a final x-y position during this step. Where subsequent positioning steps (e.g., 306, 310) may move the joining tool only in the z-direction.

At 304, in some embodiments, a first capacitance is measured between the joining tool and a conductive element extending parallel to a selected pad and/or an associated electrode to the selected pad. For example, referring back to FIG. 2A, the first capacitance is measured between joining tool 216 (e.g., between a tip 238 of joining tool 216) and conductive element 214.

Where, in some embodiments, a capacitance sensor (e.g., capacitance sensor 222 FIG. 2A) applies driving signal/s between the pad joining tool and conductive element to acquire the first capacitance measurement.

At 306, in some embodiments, the height of the joining tool above the pad (and/or flexible circuit strip) is reduced, using the first capacitance measurement. For example, to within a height of 0.5-20 mm, or 1-10 mm, or 2-10 mm from the pad. Where, in some embodiments, using the first capacitance, the height of the joining tool is reduced to a first height above the pad.

In some embodiments, steps 304 and 306 occur simultaneously and/or alternate, measurements of the first capacitance providing feedback regarding the distance. In some embodiments, movement is in a single direction e.g., height only.

In some embodiments, the height distance between the joining tool is reduced until a threshold height is reached e.g., as determined by the first capacitance reaching a threshold (e.g., as described regarding FIG. 4). Where, the first capacitance threshold may be a pre-defined threshold. In some embodiments, using the second capacitance, the height of the joining tool is reduced to a second height above the pad, the second height being smaller than the first height.

At 308, in some embodiments, a second capacitance between the specific selected pad and the joining tool is measured. For example, by capacitance sensor 208 which contacts pad 210a and joining tool tip 238 to measure capacitance therebetween. For example, by measuring capacitance between the joining tool and a portion of the flexible circuit strip directly electrically connected to the selected pad, for example, between an electrode associated with the selected pad and the joining tool (e.g., by capacitance sensor 220 which contacts electrode 126a and joining tool tip 238). The second capacitance may additionally or alternatively be measured between wire 212 extending between pad 210a and electrode 126a (not illustrated).

At 310, in some embodiments the height distance between the joining tool and the pad is reduced, using the second capacitance measurement. For example, to within 5µm-0.5 mm, or 10µm-200µm, or 10-40µm.

In some embodiments, steps 310 and 312 occur simultaneously and/or alternate, measurements of the second capacitance providing feedback regarding the distance.

In some embodiments, the joining tool is moved towards the selected pad until a threshold distance away from the pad is reached e.g., as determined by the second capacitance reaching a threshold (which may be a pre-defined threshold) and/or by a peak in capacitance being identified in the second capacitance measurement e.g., as described regarding FIG. 5.

Alternatively or additionally, in some embodiments, the joining tool is moved towards the selected pad until contact between the pad and the joining tool is identified. For example, until contact is identified in the second capacitance measurement e.g., as a drop in capacitance measured as associated with the joining tool being electrically connected to the pad (short-circuit). Optionally, in some embodiments, the first capacitance is monitored during this step in addition to the second capacitance. For example, the first capacitance being used to verify measurement/s of the second capacitance.

At 312, in some embodiments, once the distance between the joining tool and the pad is sufficiently low and/or contact between the joining tool and the tool is identified, the joining tool is activated to join a wire to the pad e.g., via a heat joining technique (e.g., where activation includes activating heating element/s e.g., supplying electrical power to the heating element/s). For example, via one or more of soldering, welding and ultrasonic joining.

Optionally, in some embodiments, the first and/or second capacitance are monitored during activation of the joining tool. For example, to provide feedback as to quality of the connection and/or temperature. Where, for example, if a low quality connection is identified, one or more parameters (e.g., relative position of the joining tool, e.g., activation parameter/s of the joining tool) may be adjusted. Where, for example, activation parameter/s (e.g., power supplied to heating element/s of the joining tool, duration of power supplied) may be adjusted based on capacitance measurement feedback regarding temperature.

At 314, optionally, in some embodiments, the joining tool is lifted from the selected pad.

In some embodiments, the method is then repeated for an additional pad and/or flexible circuit strip.

For example, starting at step 300 where an additional pad may be selected and identified e.g., optionally by acquiring additional image/s to reposition the joining tool at a general location of the additional pad.

For example, starting at step 306 e.g., where steps 300 and 302 are performed less than once per pad connection, e.g., where images are used to position the joining tool at a general location of pads of a flexible circuit strip, steps 300 and 302 being performed e.g., once per flexible circuit strip.

FIG. 4 is a simplified schematic theoretical plot of capacitance C1 with time, according to some embodiments of the disclosure.

FIG. 4, in some embodiments, illustrates the first capacitance C1 e.g., as measured between conductive element 214 (or 215) and joining tool 238.

In some embodiments, as the joining tool is brought closer to the pad, it is also brought closer to the conductive element, potentially increasing the first capacitance measured therebetween e.g., as illustrated by rising capacitance with time at section 400 of the plot. Once the joining tool has been positioned close enough to the pad, at time 402 in some embodiments, control of movement of the joining tool switches to being via the second capacitance measurement. Where, in some embodiments, arrival at the general location is identified as the first capacitance reaching threshold 406.

FIG. 5 is a simplified schematic theoretical plot of capacitance C2 with time, according to some embodiments of the disclosure.

FIG. 5, in some embodiments, illustrates the second capacitance C2, e.g., as measured between pad 210a (or electrode 242) and joining tool 238.

Where, in some embodiments, as the joining tool is moved towards the pad the capacitance measured between the two (either directly and/or as measured between the joining tool and the electrode connected to the pad) increases 500. Where, in some embodiments, time 502 corresponds to time 402 in FIG. 4. In some embodiments, sufficient proximity to the pad is determined upon the second capacitance reaching a threshold 506 and/or where a peak 501 is identified.

In some embodiments, contact between the joining tool and pad is identified by a reduction in the capacitance 504 and/or a low measured capacitance 508 measured e.g., associated with electrical short circuiting of the capacitor formed by the joining tool and pad. The short circuiting, for example, occurring as the joining tool makes electrical (e.g., and physical) contact with the pad. In some embodiments, the level of capacitance 508 measured after contact is used verify quality of the electrical and/or mechanical contact between the joining tool and pad e.g., prior to activation of the joining tool.

It should be noted that FIG. 4 and FIG. 5 are theoretical illustrative plots of expected measurements and that this disclosure should not be understood to be tied to the theory and/or particulars of the plots illustrated.

### General

As used within this document, the term "about" refers to±20%. The terms "comprises", "comprising", "includes", "including", "having" and their conjugates mean "including but not limited to". The term "consisting of' means "including and limited to".

As used herein, singular forms, for example, "a", "an" and "the" include plural references unless the context clearly dictates otherwise.

Within this application, various quantifications and/or expressions may include use of ranges. Range format should not be construed as an inflexible limitation on the scope of the present disclosure. Accordingly, descriptions including ranges should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within the stated range and/or subrange, for example, 1, 2, 3, 4, 5, and 6. Whenever a numerical range is indicated within this document, it is meant to include any cited numeral (fractional or integral) within the indicated range.

It is appreciated that certain features which are (e.g., for clarity) described in the context of separate embodiments, may also be provided in combination in a single embodiment. Where various features of the present disclosure, which are (e.g., for brevity) described in a context of a single embodiment, may also be provided separately or in any suitable sub-combination or may be suitable for use with any other described embodiment. Features described in the context of various embodiments are not to be considered essential features of those embodiments, unless the embodiment is inoperative without those elements.

Although the present disclosure has been described in conjunction with specific embodiments thereof, it is evident that many alternatives, modifications, and variations will be apparent to those skilled in the art. Accordingly, this application intends to embrace all such alternatives, modifications and variations that fall within the spirit and broad scope of the appended claims.

Citation or identification of any reference in this application should not be construed as an admission that such reference is available as prior art to the present disclosure. In addition, any priority document(s) and/or documents related to this application (e.g., co-filed) are hereby incorporated herein by reference in its/their entirety.

Where section headings are used in this document, they should not be interpreted as necessarily limiting.

### GENERAL DESCRIPTION

Following is a non-exclusive list of some exemplary embodiments of the disclosure. The present disclosure also includes embodiments which include fewer than all the features in an example and embodiments using features from multiple examples, even if not listed below.

Example 1. A method of electrically connecting a wire (244) to a connection pad (110) of a flexible circuit strip (124) using a joining tool (216), which method comprising:
receiving the flexible circuit strip (124) on a support surface (215), wherein the flexible circuit strip (124) includes a plurality of electrodes (126), a plurality of connection pads (110) and conductive traces (212) connecting each of the plurality of electrodes (124) to a connection pad of the plurality of connection pads (110);
positioning a joining tool (216) over a first connection pad (1 10a) of the flexible circuit strip (124);
monitoring a first capacitance between said joining tool (216) and a conductive layer (214) positioned under the connection pads (110), wherein said conductive layer (214) is sized, shaped, and positioned to extend over an entire footprint of said plurality of connection pads (110);
controllably lowering said joining tool (216) toward said first connection pad (210a) until the first capacitance reaches a first predefined capacitance threshold, wherein said first predefined capacitance threshold defines a first height of said joining tool (216) with respect to said flexible circuit strip (124);
monitoring a second capacitance between a first electrode (126a) electrically connected to said first connection pad (210a) and said joining tool (216);
controllably lowering said joining tool (216) from said first height until said second capacitance reaches a second predefined capacitance threshold, wherein said second predefined capacitance threshold defines a second height of said joining tool (216) with respect to said first connection pad (210a); and
activating said joining tool (216) to electrically connect said first connection pad (210a) to said wire (244).

Example 2. The method according to Example 1, wherein said first height is smaller than said second height.

Example 3. The method according to any one of Examples 1-2, wherein said conductive layer (214) is integrated into said flexible circuit strip, wherein said plurality of electrodes (126) and said plurality of connection pads (110) are disposed on an external surface (252) of said flexible circuit strip (124).

Example 4. The method according to any one of Examples 1-3, wherein said conductive layer (214) is an internal layer, said flexible circuit strip (124) including at least another layer (256) situated between said conductive layer (214) and said support surface (215).

Example 5. The method according to any one of Examples 1-4, wherein said conductive layer (214) is a conductive sheet or film that is mounted on the support surface (215) and disposed under the flexible circuit strip (124).

Example 6. The method according to Example 1, wherein said support surface is a surface of a support structure which includes conductive material which forms said conductive layer.

Example 7. The method according to any one of Examples 1-6, wherein said first height and said second height are height above a top surface (252) of said flexible circuit strip (124) measured in a direction generally away from said top surface.

Example 8. The method according to any one of Examples 1-7, comprising, prior to said controllably lowering said joining tool toward said first connection pad:
receiving one or more image of said flexible circuit strip (124); and
controllably adjusting an X-Y positioning of said joining tool with respect to said first connection pad based on the one or more image received.

Example 9. The method according to any one of Examples 1-8, comprising controllably lowering said joining tool (216) until a tip (238) of said joining tool contacts said first connection pad (210a).

Example 10. The method according to Example 9, wherein said comprising controllably lowering said joining tool (216) until a tip (238) of said joining tool contacts said first connection pad (210a) comprises identifying contact between said joining tool (216) and said first connection pad (210a) as a reduction in said second capacitance.

Example 11. The method according to any one of Examples 9-10, comprising assessing quality of said contact using said second capacitance.

Example 12. The method according to Example 11, comprising, for an additional connection pad of said plurality of connection pads (110):
repeating said controllably lowering said joining tool (216) toward said first connection pad until said first capacitance reaches said first predefined capacitance threshold; and
monitoring a third capacitance between an additional electrode electrically connected to said additional connection pad and said joining tool (216);
controllably lowering said joining tool (216) from said first height until said second capacitance reaches said second predefined capacitance threshold; and
activating said joining tool (216) to electrically connect said additional connection pad.

Example 13. The method according to any one of Examples 1-12, wherein said activating comprises activating circuitry configured to heat said joining tool.

Example 14. The method according to any one of Examples 1-13, wherein said joining tool (216) is a soldering iron or welding gun or ultrasonic joining tool.

Example 15. The method according to any one of Examples 1-14, wherein first height is less than 100 µm.

Example 16. The method according to any one of Examples 1-15, wherein said second height is less than 20µm.

Example 17. The method according to any one of Examples 1-16, wherein said joining tool (216) includes a cover comprising heat resistant and electrically conductive material.

Example 18. The method according to Example 17, wherein said cover comprises gold.

Example 19. A controller (218) for controlling electrical connecting of a wire (244) to a connection pad (110) of a flexible circuit strip (124) using a joining tool (216) moveable by one or more actuator (232), where said flexible circuit strip (124) includes a plurality of electrodes (126), a plurality of connection pads (110) and conductive traces (212) connecting each of the plurality of electrodes (126) to a connection pad of the plurality of connection pads (110), which controller (218) comprising circuitry configured to:
instruct said one or more actuator (232) to position said joining tool (216) over a first connection pad of said plurality of connection pads (110);
receive measurement of a first capacitance between said joining tool (216) and a conductive layer (214) positioned under the connection pads (110), wherein said conductive layer (214) is sized, shaped, and positioned to extend over an entire footprint of said plurality of connection pads (110);
instruct said one or more actuator (232) to lower said joining tool (216) toward said first connection pad (210a) until said first capacitance reaches a first predefined capacitance threshold, wherein said first predefined capacitance threshold defines a first height of said joining tool (216) with respect to said flexible circuit strip (124);
receive measurement of a second capacitance between an electrode (126a) electrically connected to said first connection pad (210a) and said joining tool (216);
instruct said one or more actuator (232) to lower said joining tool (216) from said first height until said second capacitance reaches a second predefined capacitance threshold, wherein said second predefined capacitance threshold defines a second height of said joining tool with respect to said first connection pad (210a); and
instruct said joining tool (216) to activate to electrically connect said pad (210a) to said wire (244).

Example 20. The controller according to Example 19, wherein said controller (218) is configured to generate driving signals for measurement of said first capacitance and for measurement of said second capacitance.

Example 21. A system (200) for connecting a wire (244) to a connection pad (110) of a flexible circuit strip (124) including a plurality of electrodes (126), a plurality of connection pads (110) and conductive traces (212) connecting each of the plurality of electrodes (126) to a connection pad of the plurality of connection pads (110) which system comprising:
a joining tool (216);
one or more actuator (232) configured to control a height between said joining tool (216) and a flexible circuit strip support (215) configured to receive said flexible circuit strip (124);
one or more capacitance sensor (208, 220, 222) configured to:
   measure a first capacitance between said joining tool (216) and a conductive layer (214) positioned under the connection pads (110), wherein said conductive layer (214) is sized, shaped, and positioned to extend over an entire footprint of said plurality of connection pads (110); and
   measure a second capacitance between said joining tool (216) and a first electrode (126a) electrically connected to said first connection pad (210a);
one or more electrical connector configured to electrically connect said one or more capacitance sensor to said joining tool, said conductive layer, and said first electrode;
a controller (218) configured to:
   instruct said one or more actuator (232) to position said joining tool (216) over a first connection pad (210a) of said plurality of connection pads (110);
   receive measurement of a first capacitance between said joining tool (216) and a conductive layer (214) in close proximity to or part of said flexible circuit strip (124), where said plurality of connection pads (110) are disposed between said conductive element (214) and said joining tool (216);
   instruct said one or more actuator (232) to lower said joining tool (216) toward said first connection pad until said first capacitance reaches a first predefined capacitance threshold, wherein said first predefined capacitance threshold defines a first height of said joining tool (216) with respect to said flexible circuit strip;
   receive measurement of a second capacitance between an electrode (126a) electrically connected to said first connection pad (210a) and said joining tool (216);
   instruct said one or more actuator (232) to lower said joining tool (216) from said first height until said second capacitance reaches a second predefined capacitance threshold, wherein said second predefined capacitance threshold defines a second height of said joining tool (216) with respect to said first connection pad (210a); and
   instruct said joining tool (216) to activate to electrically connect said pad (210a) to said wire (244).

Example 22. The system according to Example 21, comprising a camera (226) configured to acquire one or more image of said flexible circuit strip (124), wherein said controller (218) is configured to:
receive said one or more image of said flexible circuit strip;
identify said first connection pad (210a) in said one or more image;
determine a spatial relationship between said joining tool (216) and said first connection pad (210a) using said one or more image; and
instruct said one or more actuator (232) to position said joining tool (216) over said first connection pad (210a) using said spatial relationship.

Example 23. The system according to any one of Examples 21-22, wherein said joining tool includes a cover comprising heat resistant and electrically conductive material.

Example 24. The system according to Example 23, wherein said one or more electrical connector connects to said joining tool (216) via said joining tool cover.

## Claims

1. A method of electrically connecting a wire (244) to a connection pad (110) of a flexible circuit strip (124) using a joining tool (216), which method comprising:
receiving the flexible circuit strip (124) on a support surface (215), wherein the flexible circuit strip (124) includes a plurality of electrodes (126), a plurality of connection pads (110) and conductive traces (212) connecting each of the plurality of electrodes (124) to a connection pad of the plurality of connection pads (110);
positioning a joining tool (216) over a first connection pad (1 10a) of the flexible circuit strip (124);
monitoring a first capacitance between said joining tool (216) and a conductive layer (214) positioned under the connection pads (110), wherein said conductive layer (214) is sized, shaped, and positioned to extend over an entire footprint of said plurality of connection pads (110);
controllably lowering said joining tool (216) toward said first connection pad (210a) until the first capacitance reaches a first predefined capacitance threshold, wherein said first predefined capacitance threshold defines a first height of said joining tool (216) with respect to said flexible circuit strip (124);
monitoring a second capacitance between a first electrode (126a) electrically connected to said first connection pad (210a) and said joining tool (216);
controllably lowering said joining tool (216) from said first height until said second capacitance reaches a second predefined capacitance threshold, wherein said second predefined capacitance threshold defines a second height of said joining tool (216) with respect to said first connection pad (210a); and
activating said joining tool (216) to electrically connect said first connection pad (210a) to said wire (244).

2. The method according to claim 1, wherein said conductive layer (214) is integrated into said flexible circuit strip, wherein said plurality of electrodes (126) and said plurality of connection pads (110) are disposed on an external surface (252) of said flexible circuit strip (124).

3. The method according to any one of claims 1-2, wherein said conductive layer (214) is an internal layer, said flexible circuit strip (124) including at least another layer (256) situated between said conductive layer (214) and said support surface (215).

4. The method according to any one of claims 1-3, wherein said conductive layer (214) is a conductive sheet or film that is disposed between the support surface (215) and an underside of the flexible circuit strip (124).

5. The method according to claim 1, wherein one or more of:
said support surface (215) is a surface of a support structure which includes conductive material which forms said conductive layer; and
said first height and said second height are height above a top surface (252) of said flexible circuit strip (124) measured in a direction generally away from said top surface (252).

6. The method according to any one of claims 1-5, comprising one or more of:
prior to said controllably lowering said joining tool toward said first connection pad: receiving one or more image of said flexible circuit strip (124) and controllably adjusting an X-Y positioning of said joining tool with respect to said first connection pad (210a) based on the one or more image received; and
controllably lowering said joining tool (216) until a tip (238) of said joining tool contacts said first connection pad (210a).

7. The method according to claim 6, wherein said comprising controllably lowering said joining tool (216) until a tip (238) of said joining tool contacts said first connection pad (210a) comprises identifying contact between said joining tool (216) and said first connection pad (210a) as a reduction in said second capacitance.

8. The method according to any one of claims 6-7, comprising one or more of:
assessing quality of said contact using said second capacitance; and for an additional connection pad of said plurality of connection pads (110):
repeating said controllably lowering said joining tool (216) toward said first connection pad (210a) until said first capacitance reaches said first predefined capacitance threshold; and
monitoring a third capacitance between an additional electrode electrically connected to said additional connection pad and said joining tool;
controllably lowering said joining tool (216) from said first height until said second capacitance reaches said second predefined capacitance threshold; and
activating said joining tool (216) to electrically connect said additional connection pad.

9. The method according to any one of claims 1-8, wherein one or more of:
said activating comprises activating circuitry configured to heat said joining tool (216);
said joining tool (216) is a soldering iron or welding gun or ultrasonic joining tool;
said first height is smaller than said second height; said first height is less than 100 µm; and
said second height is less than 20µm.

10. The method according to any one of claims 1-9, wherein said joining tool (216) includes a cover comprising heat resistant and electrically conductive material.

11. A controller (218) for controlling electrical connecting of a wire (244) to a connection pad (110) of a flexible circuit strip (124) using a joining tool (216) moveable by one or more actuator (232), where said flexible circuit strip (124) includes a plurality of electrodes (126), a plurality of connection pads (110) and conductive traces (212) connecting each of the plurality of electrodes (126) to a connection pad of the plurality of connection pads (110), which controller (218) comprising circuitry configured to:
instruct said one or more actuator (232) to position said joining tool (216) over a first connection pad of said plurality of connection pads (110);
receive measurement of a first capacitance between said joining tool (216) and a conductive layer (214) positioned under the connection pads (110), wherein said conductive layer (214) is sized, shaped, and positioned to extend over an entire footprint of said plurality of connection pads (110);
instruct said one or more actuator (232) to lower said joining tool (216) toward said first connection pad (210a) until said first capacitance reaches a first predefined capacitance threshold, wherein said first predefined capacitance threshold defines a first height of said joining tool (216) with respect to said flexible circuit strip (124);
receive measurement of a second capacitance between an electrode (126a) electrically connected to said first connection pad (210a) and said joining tool (216);
instruct said one or more actuator (232) to lower said joining tool (216) from said first height until said second capacitance reaches a second predefined capacitance threshold, wherein said second predefined capacitance threshold defines a second height of said joining tool with respect to said first connection pad (210a); and
instruct said joining tool (216) to activate to electrically connect said pad (210a) to said wire (244).

12. The controller according to claim 11, wherein said controller (218) is configured to generate driving signals for measurement of said first capacitance and for measurement of said second capacitance.

13. A system (200) for connecting a wire (244) to a connection pad (110) of a flexible circuit strip (124) including a plurality of electrodes (126), a plurality of connection pads (110) and conductive traces (212) connecting each of the plurality of electrodes (126) to a connection pad of the plurality of connection pads (110) which system comprising:
a joining tool (216);
one or more actuator (232) configured to control a height between said joining tool (216) and a flexible circuit strip support (215) configured to receive said flexible circuit strip (124);
one or more capacitance sensor (208, 220, 222) configured to:
measure a first capacitance between said joining tool (216) and a conductive layer (214) positioned under the connection pads (110), wherein said conductive layer (214) is sized, shaped, and positioned to extend over an entire footprint of said plurality of connection pads (110); and
measure a second capacitance between said joining tool (216) and a first electrode (126a) electrically connected to said first connection pad (210a);
one or more electrical connector configured to electrically connect said one or more capacitance sensor (208, 220, 222) to said joining tool (216), said conductive layer (214), and said first electrode (126a);
a controller (218) configured to:
instruct said one or more actuator (232) to position said joining tool (216) over a first connection pad (210a) of said plurality of connection pads (110);
receive measurement of a first capacitance between said joining tool (216) and a conductive layer (214) in close proximity to or part of said flexible circuit strip (124), where said plurality of connection pads (110) are disposed between said conductive element (214) and said joining tool (216);
instruct said one or more actuator (232) to lower said joining tool (216) toward said first connection pad until said first capacitance reaches a first predefined capacitance threshold, wherein said first predefined capacitance threshold defines a first height of said joining tool (216) with respect to said flexible circuit strip;
receive measurement of a second capacitance between an electrode (126a) electrically connected to said first connection pad (210a) and said joining tool (216);
instruct said one or more actuator (232) to lower said joining tool (216) from said first height until said second capacitance reaches a second predefined capacitance threshold, wherein said second predefined capacitance threshold defines a second height of said joining tool (216) with respect to said first connection pad (210a); and
instruct said joining tool (216) to activate to electrically connect said pad (210a) to said wire (244).

14. The system according to claim 13, comprising a camera (226) configured to acquire one or more image of said flexible circuit strip (124), wherein said controller (218) is configured to:
receive said one or more image of said flexible circuit strip;
identify said first connection pad (210a) in said one or more image;
determine a spatial relationship between said joining tool (216) and said first connection pad (210a) using said one or more image; and
instruct said one or more actuator (232) to position said joining tool (216) over said first connection pad (210a) using said spatial relationship.

15. The system according to any one of claims 13-14, wherein said joining tool (216) includes a cover comprising heat resistant and electrically conductive material; and
wherein said one or more electrical connector connects to said joining tool via said joining tool cover.
